# EUROPÄISCHE PATENTSCHRIFT

(11) **EP 0 663 911 B1**
(45) Veröffentlichungstag und Bekanntmachung des Hinweises auf die Patenterteilung: **05.02.1997**
(21) Anmeldenummer: 93921925.9
(22) Anmeldetag: 06.10.1993
(51) Int. Cl.: C07D 311/18, C07D 311/16, C07D 405/06, A61K 31/37, A61K 31/445

(54) **NEUE BENZOPYRANONE, VERFAHREN ZU IHRER HERSTELLUNG UND VERWENDUNG**
BENZOPYRANONES, METHOD OF PREPARING THEM AND THEIR USE
NOUVELLES BENZOPYRANONES, LEUR PROCEDE DE PREPARATION ET LEUR UTILISATION

(30) Priorität: 08.10.1992 DE 4233963
(43) Veröffentlichungstag der Anmeldung: 26.07.1995
(73) Patentinhaber: Dr. Willmar Schwabe GmbH & Co., D-76227 Karlsruhe (DE)
(72) Erfinder: NÖLDNER, Michael, D-76344 Eggenstein (DE); CHATTERJEE, Shyam, Sunder, D-76139 Karlsruhe (DE); HAUER, Hermann, D-76228 Karlsruhe (DE)
(74) Vertreter: Bunke, Holger, Dr.rer.nat. Dipl.-Chem.
(86) Internationale Anmeldenummer: EP9302742
(87) Internationale Veröffentlichungsnummer: WO9408985

(56) Entgegenhaltungen:
- DE-A- 2 448 257
- US-A- 3 538 098
- US-A- 3 930 003
- JOURNAL OF THE INDIAN CHEMICAL SOCIETY Bd. 67, Nr. 6 , Juni 1990 , CALCUTTA Seiten 482 - 484 RAJEEV VYAS ET AL 'Synthesis,characterisation and testing of some new 7,8-dimethoxy-3-substituted-amino methyl-4-methylcoumarins as possible antibacterial agents' in der Anmeldung erwähnt

## Beschreibung

Die Erfindung betrifft Benzopyranone, deren Grundstruktur sich von Cumarin ableiten läßt, Verfahren zur Herstellung dieser Verbindungen sowie Arzneimittel, die diese Verbindungen enthalten.

Das Zentralnervensystem (ZNS) von Säugetieren besitzt hohe Konzentrationen von exzitatorischen Aminosäuren (EAS) wie Glutamat, Aspartat und Homocysteat, die als Neurotransmitter fungieren, welche mit spezifischen Rezeptoren zusammenwirken.

Die drei am besten charakterisierten Rezeptor-Typen sind die nach ihren selektiven Agonisten benannten N-Methyl-D-aspartat- (NMDA), Kainat- (KA) und Quisqualat- (QA) Rezeptoren. Alle drei Rezeptoren können durch Glutamat und Aspartat aktiviert werden. Es ist bekannt, daß als Folge cerebraler Ischämien Glutamat in größerer Menge freigesetzt wird, welches u. a. am NMDA-Rezeptorkomplex bindet und zu einem vermehrten Calciumeinstrom sowie zu einer erhöhten Freisetzung intrazellulären Calciums in den neuronalen Zellen führt. Der NMDA-Rezeptorkomplex beinhaltet u. a. Bindungsstellen für Glutamat, Glycin, Phencyclidin, Mg²⁺ und Zn²⁺. Da eine Reihe von pharmakologischen Befunden darauf hindeutet, daß Modulatoren der NMDA-Rezeptor-vermittelten Neurotransmission die NMDA-vermittelte Zytotoxizität beeinflussen können, wurden bereits verschiedene selektive NMDA-Antagonisten im Hinblick auf ihre mögliche neuroprotektive Wirkung untersucht (vgl. G. L. Collingridge, R. A. J. Lester: "Excitatory Amino Acid Receptors in the Vertebrate Central Nervous System", Pharmacol. Rev. 40, No. 2, S. 143 - 210 (1989); L. Turski: "N-Methyl-D-aspartat-Rezeptorkomplex", Arzneim.-Forsch. / Drug Res. 40 (I), Nr. 5, S. 511 - 514 (1990)). Wegen unerwünschter Nebenwirkungen der bekannten NMDA-Antagonisten besteht weiterhin ein starkes Bedürfnis nach der Bereitstellung neuer Verbindungen mit NMDA-antagonistischer Wirkung, die geringere Nebenwirkungen oder ein anderes Wirkungsspektrum zeigen.

Der Erfindung liegt deshalb die Aufgabe zugrunde, neue Verbindungen zu schaffen, die eine möglichst geringe Toxizität, aber dennoch NMDA-antagonistische Wirkung besitzen und als Arzneimittelwirkstoffe, insbesondere in der Therapie chronischer neurodegenerativer Erkrankungen, verwendet werden können, um durch Ischämie / Trauma oder durch andere pathologische Veränderungen verursachte Neurodegenerationen im ZNS und das Auftreten von Konvulsionen zu verhindern oder doch mindestens zu verringern.

Diese Aufgabe wird durch die Bereitstellung der erfindungsgemäßen Verbindungen und Verfahren sowie durch die Verwendung dieser Verbindungen als neuroprotektiv, antikonvulsiv und antiepileptisch wirksame Arzneimittel gelöst. Die erfindungsgemäßen Verbindungen besitzen daneben noch bemerkenswerte antidepressive, nootropische, antipsychotische und anxiolytische Eigenschaften.

Gegenstand der Erfindung sind somit:

2H-1-Benzopyran-2-one der allgemeinen Formel I, 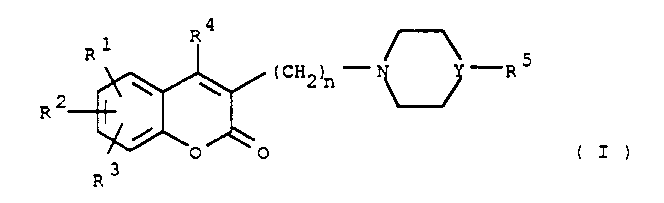 worin
R¹ einen Hydroxyrest, einen Alkoxyrest mit 1 bis 5 C-Atomen, einen Cycloalkoxyrest mit 4 bis 6 C-Atomen oder einen Alkyl- oder Arylsulfonyloxyrest R⁶-SO₂O-,
R² und R³ unabhängig voneinander Wasserstoffatome, Hydroxyreste, Alkoxyreste mit 1 bis 4 C-Atomen oder Cycloalkoxyreste mit 4 bis 6 C-Atomen,
R⁴ ein Wasserstoffatom, eine Alkylgruppe mit 1 bis 4 C-Atomen oder einen Phenylrest,
Y ein Stickstoffatom, eine CH-Gruppe oder eine COH-Gruppe,
R⁵ einen Phenyl-, Naphthyl-, Pyridinyl- oder Pyrimidinyl-Rest, der gegebenenfalls mit jeweils ein oder zwei C₁-C₅-Alkylgruppen, mit jeweils ein oder zwei Halogenatomen, mit Halogen und gleichzeitig C₁-C₅-Alkyl, mit Perfluoralkyl mit 1 bis 3 C-Atomen, C₁-C₅-Alkoxy, Hydroxy, Methylendioxy oder Nitro substituiert ist,
R⁶ einen Alkylrest mit 1 bis 5 C-Atomen, einen Cycloalkylrest mit 4 bis 6 C-Atomen oder einen Phenylrest, der gegebenenfalls mit jeweils ein oder zwei C₁-C₅-Alkylgruppen, mit jeweils ein oder zwei Halogenatomen oder mit Perfluoralkyl mit 1 bis 3 C-Atomen substituiert ist, und
n eine ganze Zahl von 1 - 4
bedeuten, sowie deren Additionsverbindungen mit physiologisch verträglichen Säuren, jedoch mit Ausnahme von 7,8-Dimethoxy4-methyl-3-[(4-phenyl-1-piperazinyl)methyl]-2H-1-benzopyran-2-on.

Die erfindungsgemäßen Verbindungen sind neu. Bereits bekannt ist die Verbindung 7,8-Dimethoxy-4-methyl-3-[(4-phenyl-1-piperazinyl)methyl]-2H-1-benzopyran-2-on, die jedoch nur auf ihre antibakterielle Wirkung geprüft wurde (R. Vyas, S. Bapat, R. H. Mehta, J. Indian Chem. Soc. 67, No. 6, S. 482 - 484 (1990)).

Ferner sind Verbindungen bekannt, bei denen der Cumarinrest und der Piperazinrest durch eine gegebenenfalls O-acylierte 2-Hydroxypropylenkette verbunden sind (DE-C3-1668877). In DE-A-1670395, DE-A-1670465 und DE-A-1670468 werden Verbindungen beschrieben, bei denen der Piperazin-Stickstoff einen substituierten Benzoyloxyalkylrest trägt. Strukturell noch weiter entfernt von den erfindungsgemäßen Verbindungen der allgemeinen Formel I sind substituierte 7-(Aminocarbonylamino)cumarinderivate (DE-A-2108185, DE-A-2530405 und DE-A-2543945). In allen genannten Patentschriften wird nur eine koronardilatorische Wirksamkeit der dort beanspruchten Verbindungen erwähnt.

Weitere bekannte Verbindungen sind 7-(Aminothiocarbonylamino)-cumarinderivate mit koronardilatorischer und teilweise zusätzlicher analgetischer, sedativer und antiphlogistischer Wirksamkeit (DE-A1-2448257). Ferner sind Sulfonsäureester von Hydroxycumarinen bekannt, denen jedoch die Piperazin- bzw. Piperidinreste fehlen und von denen nur eine antidepressive Wirkung bekannt ist (EP-B-111746).

Im Hinblick auf diesen Stand der Technik war es für den Fachmann überraschend und in keiner Weise vorhersehbar, daß die erfindungsgemäßen Verbindungen der allgemeinen Formel I eine NMDA-antagonistische und neuroprotektive Wirkung aufweisen.

Erfindungsgemäß bevorzugte Verbindungen der allgemeinen Formel I sind solche, bei denen
R¹ einen Hydroxy-, Methoxy-, Ethoxy-, Propyloxy oder Ethansulfonyloxyrest,
R² und R³ unabhängig voneinander Wasserstoffatome, Hydroxy- oder Alkoxyreste mit 1 bis 3 C-Atomen,
R⁴ eine Methylgruppe
Y ein Stickstoffatom, eine CH-Gruppe oder eine COH-Gruppe,
R⁵ einen ggf. mit Hydroxy, Methoxy, Ethoxy, Methyl, Fluor oder Trifluormethyl substituierten Phenylrest,
n=2
bedeuten, sowie deren Additionsverbindungen mit physiologisch verträglichen Säuren.

Bei dem erfindungsgemäßen Verfahren zur Herstellung der Verbindungen der allgemeinen Formel I wird eine Verbindung der allgemeinen Formel II, 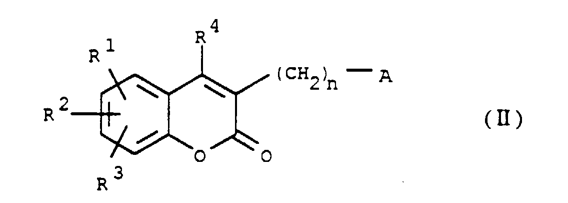 worin R¹ bis R⁴ und n die oben angegebenen Bedeutungen haben und A eine Abgangsgruppe ist, die ausgewählt ist aus der Gruppe Chlor, Brom, Iod, Akylsulfonyloxy, Trifluormethansulfonyloxy, gegebenfalls mit Alkyl, Nitro oder Halogen substituiertes Phenylsulfonyloxy, mit einer Verbindung der allgemeinen Formel III 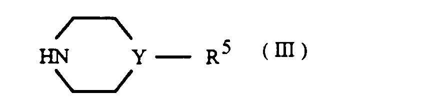 umgesetzt, in der Y und R⁵ die oben angegebenen Bedeutungen haben, wobei die Verbindung der allgemeinen Formel III auch in Form ihres Hydrochlorids oder eines anderen Säureadditionssalzes vorliegen kann. Darauf werden die erhaltenen Produkte gegebenenfalls in ihre physiologisch verträglichen Säureadditionsverbindungen übergeführt.

Die Umsetzungen werden in an sich bekannter Weise durchgeführt. So wird z. B. in DE-A-1670468 die Reaktion von 3-(2-Bromethyl)-4-methyl-6,7-dimethoxy-2H-1-benzopyran-2-on mit 1-(2-Hydroxyethyl)piperazin beschrieben. Zur Bindung der entstehenden Säure HA, worin A eine der vorstehend definierten Abgangsgruppen ist, finden diese Reaktionen in Gegenwart einer Base wie z. B. eines Alkali- oder Erdalkalicarbonates, - hydrogencarbonates, -hydrids, -alkoholats, -hydroxyds oder eines tertiären Amins statt, wobei vorzugsweise Alkalicarbonate und -hydrogencarbonate zum Einsatz kommen. Die Verwendung eines Überschusses des Reaktionspartners III als Base ist ebenfalls möglich. Die Umsetzungen werden vorteilhaft in Gegenwart von gegenüber den Reaktanden inerten Lösungsmitteln durchgeführt. Gut geeignet sind hierfür Alkanole, aromatische Lösungsmittel wie Toluol, Xylol, Chlorbenzol usw., oder dipolare aprotische Lösungsmittel wie aliphatische oder cycloaliphatische Ether, Carbonsäuredialkylamide, Tetraalkylharnstoffe, Ketone und Sulfoxide. Bevorzugt sind Alkanole mit 1 bis 5 C-Atomen und Dimethylformamid. Gegebenenfalls können 0.02 bis 0.5 Äquivalente eines Alkali- oder Erdalkaliiodids, vorzugsweise 0.05 bis 0.2 Äquivalente Kaliumiodid, als Katalysator zugesetzt werden. Die Umsetzung kann bei einer Temperatur durchgeführt werden, die zwischen Raumtemperatur und 130°C, bevorzugt jedoch zwischen Raumtemperatur und 100°C, bzw., bei niedersiedenden Lösungsmitteln, in der Nähe des Siedepunkts liegt. Oxidative Nebenprodukte lassen sich durch Arbeiten unter einer Schutzgasatmosphäre, beispielsweise Stickstoff oder Argon, vermeiden. Die Reaktionen werden unter Normaldruck oder in geschlossenen Gefäßen bei Drücken bis zu etwa 10⁷ Pa (100 bar) durchgeführt.

Die Herstellung derjenigen Verbindungen der allgemeinen Formel I, bei denen R¹ ein Alkyl- oder Arylsulfonyloxyrest ist, kann auch erfolgen durch Umsetzung der entsprechenden Verbindung der allgemeinen Formel I, bei der R¹ ein Hydroxyrest ist, mit einem Sulfonsäurehalogenid. Dazu setzt man den Alkohol der allgemeinen Formel I in an sich bekannter Weise mit einem Sulfonsäurehalogenid R⁶SO₂Hal mit der oben angegebenen Bedeutung für R⁶ um. Als Lösungsmittel eignen sich z. B. Aromaten wie Toluol, Xylol usw., oder dipolare aprotische Lösungsmittel wie aliphatische oder cycloaliphatische Ether, Carbonsäuredialkylamide, Tetraalkylharnstoffe, Ketone, Sulfoxide und halogenierte Alkane. Bevorzugt werden jedoch Wasser oder niedere Alkohole eingesetzt. Zur Bindung der entstehenden Halogenwasserstoffsäure setzt man eine geeignete Base wie z. B. ein Alkali- oder Erdalkalicarbonat oder ein tertiäres oder aromatisches Amin zu, vorzugsweise jedoch ein Alkali- oder Erdalkalihydroxyd. Die Reaktionstemperatur kann zwischen -30°C und +80°C, bevorzugt jedoch zwischen 0°C und +50°C, liegen. Oxidative Nebenprodukte lassen sich durch Arbeiten unter Schutzgasatmosphäre, beispielsweise Stickstoff oder Argon, vermeiden.

Die für die Herstellung der erfindungsgemäßen Verbindungen der allgemeinen Formel I benötigten reaktiven Zwischenprodukte der allgemeinen Formel II, 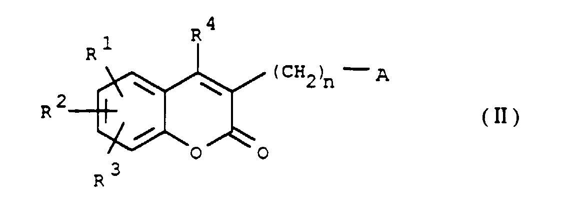 worin R¹ bis R⁴ und n die oben angegebenen Bedeutungen haben und A eine Abgangsgruppe ist, die ausgewählt ist aus der Gruppe Chlor, Brom, Iod, Alkylsulfonyloxy, Trifluormethylsulfonyloxy, gegebenfalls mit Alkyl, Nitro oder Halogen substituiertes Phenylsulfonyloxy, werden aus den entsprechenden Alkoholen der allgemeinen Formel IV dargestellt.

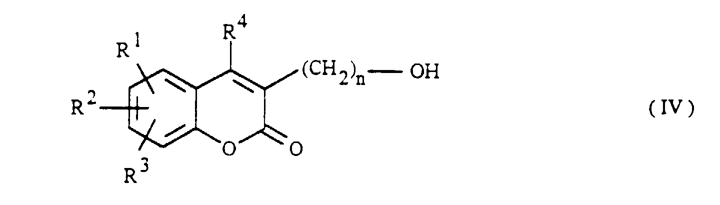

Dazu setzt man entweder einen Alkohol der allgemeinen Formel IV in an sich bekannter Weise
a) mit einem Säurehalogenid wie z. B. Thionylchlorid, Thionylbromid, Phosphoroxychlorid usw. oder mit einer Säure wie Bromwasserstoff- oder Iodwasserstoffsäure, wobei Halogenide der allgemeinen Formel II erhalten werden, oder
b) mit einem Sulfonsäurechlorid wie z. B. mit Alkylsulfonyl-, Trifluormethylsulfonyl- oder mit gegebenfalls mit Alkyl, Nitro oder Halogen substituiertem Phenylsulfonylchlorid um, wobei Sulfonate der allgemeinen Formel II erhalten werden.

Die Reaktionen unter a) finden in einem gegenüber den Reaktionspartnern inerten Lösungsmittel, bevorzugt jedoch in überschüssigem Säurehalogenid als Lösungsmittel statt. Bei den Reaktionen unter b) verwendet man ebenfalls gegenüber den Reaktionspartnern inerte Lösungsmittel. Gut geeignet sind hierfür aromatische Lösungsmittel wie Toluol, Xylol usw., oder dipolare aprotische Lösungsmittel wie aliphatische oder cycloaliphatische Ether, Carbonsäuredialkylamide, Tetraalkylharnstoffe, Ketone, Sulfoxide und halogenierte Alkane. Bevorzugt sind bei b) halogenierte Alkane wie Chloroform oder Dichlormethan. Ferner setzt man bei b) zur Bindung der entstehenden Halogenwasserstoffsäure eine geeignete Base wie z. B. ein Alkali- oder Erdalkalicarbonat oder ein tertiäres oder aromatisches Amin zu, wobei vorzugsweise tertiäre Amine wie Triethylamin zum Einsatz kommen. Bei a) und b) kann die Reaktionstemperatur zwischen -30°C und +80°C, bevorzugt jedoch zwischen 0°C und +50°C, liegen. Oxidative Nebenprodukte lassen sich durch Arbeiten unter Schutzgasatmosphäre, beispielsweise Stickstoff oder Argon, vermeiden.

Zwischenprodukte der allgemeinen Formel II, bei denen R¹ ein Alkyl- oder Arylsulfonyloxyrest ist, kann man aus der entsprechenden Verbindung der allgemeinen Formel II, bei der R¹ ein Hydroxyrest ist, herstellen. Dazu setzt man diese mit einem Sulfonsäurehalogenid um, wie oben bei der entsprechenden Reaktion der Endprodukte der allgemeinen Formel I beschrieben.

Die Zwischenprodukte IV werden aus den entprechend substituierten Phenolen V 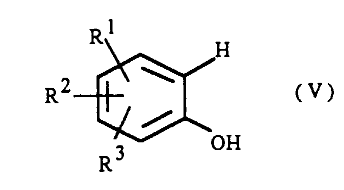 hergestellt, worin R¹, R² und R³ die oben angegebenen Bedeutungen haben. Falls einer oder mehrere der Reste R¹, R² und R³ eine Hydroxygruppe darstellt, kann nach der nachfolgend näher beschriebenen Cyclisierungsreaktion eine Alkylierung dieser Hydroxygruppen durchgeführt werden.

Zur Herstellung der Zwischenprodukte IV werden die Phenole der allgemeinen Formel V mit β-Ketocarbonsäureestern der allgemeinen Formel VI, 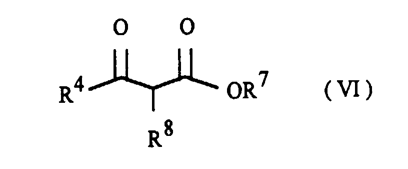 in der R⁴ die oben angegebene Bedeutung hat, R⁷ ein Niederalkylrest und R⁸ ein Hydroxyalkylenrest (CH₂)ₙOH und oder R⁷ und R⁸ gemeinsam eine Alkylenkette (CH₂)ₙ mit der oben angegebenen Bedeutung für n ist, in an sich bekannter Weise in Gegenwart eines sauren Katalysators umgesetzt. Als sauren Katalysator verwendet man Mineral- oder Lewissäuren ohne Lösungsmittel oder in gegenüber der Reaktion inerten Lösungsmitteln wie z. B. Alkoholen oder Eisessig, vorzugsweise 50- bis 100%ige Schwefelsäure ohne weiteres Lösungsmittel. Die Reaktion wird bei 0°C bis 60°C, vorzugsweise bei 0°C bis Raumtemperatur durchgeführt.

Gegenstand der Erfindung sind ferner Arzneimittel, die als Wirkstoff eine oder mehrere der erfindungsgemäßen Verbindungen der allgemeinen Formel I sowie gegebenenfalls zusätzlich pharmakologisch inerte Hilfsstoffe wie zum Beispiel Wasser, pflanzliche Öle, Polyethylenglykole, Glycerinester, Gelatine, Kohlenhydrate wie Lactose oder Stärke, Magnesiumstearat, Talk, Vaseline®, Konservierungsmittel, Netzmittel, Emulgatoren, physiologisch unbedenkliche Salze, Puffersubstanzen, Farbstoffe, Geschmacksstoffe und Aromastoffe enthalten. Die Wahl der Begleitstoffe hängt von der gewünschten Applikationsform wie z. B. Tabletten, Dragees, Säfte, Ampullen, Suppositorien, Salben oder Sprays ab. Die erfindungsgemäßen Verbindungen können auch im Gemisch mit anderen bekannten Wirkstoffen verabreicht werden.

In den nachfolgenden Beispielen sind die erfindungsgemäßen Verbindungen, die Verfahren zu ihrer Herstellung sowie die pharmakologischen Versuchsergebnisse näher beschrieben.

Die im nachfolgenden Text verwendete Abkürzung TBME bedeutet tert.-Butyl-Methylether.

### I. Beispiele 1 bis 25 für Endprodukte der allgemeinen Formel I

Zur Herstellung der in den nachfolgenden Beispielen 1 bis 25 näher beschriebenen Verbindungen wurden folgende Verfahren benutzt:

Verfahren A: Das entsprechend mit R¹ bis R⁴ substituierte (Methansulfonyloxy)alkyl- oder Chloralkyl-2H-1-Benzopyran-2-on der allgemeinen Formel II wird mit 1.0 bis 1.2 Äquivalenten des gewünschten mit R⁵ substituierten Piperidins oder Piperazins der allgemeinen Formel III in Form der Base oder des Hydrochlorids, mit 1.5 bis 3 Äquivalenten Kaliumhydrogencarbonat und mit 0.1 bis 0.4 Äquivalenten Kaliumiodid in Dimethylformamid 3 h bis 70 d unter Stickstoff bei 50 - 70°C gerührt. Man entfernt das Lösungsmittel im Vakuum, nimmt in Ethylacetat/ Wasser oder Chloroform/Wasser auf, wobei unter Umständen ein Teil des Produkts nicht in Lösung geht und sofort abgesaugt werden kann, wäscht mit Wasser, trocknet über Natriumsulfat, entfernt erneut das Lösungsmittel und reinigt durch Umkristallisation oder säulenchromatographisch über Kieselgel.

Verfahren B: Das entsprechend mit R¹ bis R⁴ substituierte (Methansulfonyloxy)alkyl- oder Chloralkyl-2H-1-Benzopyran-2-on der allgemeinen Formel II wird mit 2.0 bis 2.7 Äquivalenten des gewünschten mit R⁵ substituierten Piperidins oder Piperazins der allgemeinen Formel III und mit 0.1 bis 0.2 Äquivalenten Kaliumiodid in Dimethylformamid 15 h bis 7 d unter Stickstoff bei 40 bis 90°C gerührt. Man entfernt das Lösungsmittel im Vakuum und nimmt in Chloroform oder Ethylacetat auf. Falls das Produkt nicht kristallin anfällt und abgesaugt werden kann, wäscht man gegebenenfalls mit verdünnter Natronlauge und mit Wasser, trocknet über Natriumsulfat und entfernt erneut das Lösungsmittel. Man reinigt durch Umkristallisation oder säulenchromatographisch über Kieselgel.

Verfahren C: Zu einer Lösung der Base in Ethanol, Isopropanol, 2-Butanon oder Chloroform gibt man ein Äquivalent Fumarsäure, gelöst in Ethanol oder Isopropanol. Das Fumarat fällt sofort oder beim Einengen kristallin an und wird umkristallisiert. Soweit in den Beispielen nichts anderes beschrieben wird, wird jeweils 1.0 C₄H₄O₄ addiert.

### Beispiel 1: 7-Methoxy-3-{2-[4-(2-methoxyphenyl)-1-piperazinyl]ethyl}-4-methyl-2H-1-benzopyran-2-on

Verfahren A; Ausgangsmaterialien: 3-[2-(Methansulfonyloxy)ethyl]-7-methoxy-4-methyl-2H-1-benzopyran-2-on (Beispiel 26) und 1-(2-Methoxyphenyl)piperazin; Ausbeute 29 %; Schmp. 129 - 130°C (aus Ethanol/TBME).

Fumarat (x 0.5 C₄H₄O₄): Verfahren C; Ausbeute 97 %; Schmp. 182 - 185°C (aus Ethanol).

### Beispiel 2: 6-Hydroxy-7-methoxy-3-{2-[4-(2-methoxyphenyl)-1-piperazinyl]ethyl}-4-methyl-2H-1-benzopyran-2-on

Verfahren A; Ausgangsmaterialien: 3-(2-Chlorethyl)-6-hydroxy-7-methoxy-4-methyl-2H-1-benzopyran-2-on (Beispiel 27) und 1-(2-Methoxyphenyl)piperazin; Ausbeute 14 %; Schmp. 235 - 237°C (aus Ethanol/Chloroform).

### Beispiel 3: 6,7-Dimethoxy-4-methyl-3-[2-(4-phenyl-1-piperazinyl)ethyl]-2H-1-benzopyran-2-on

Verfahren B; Ausgangsmaterialien: 3-[2-(Methansulfonyloxy)ethyl]-6,7-dimethoxy-4-methyl-2H-1-benzopyran-2-on (Beispiel 28) und 1-Phenylpiperazin; Ausbeute 43 %; Schmp. 169 - 172°C (aus Isopropanol).

Fumarat (x 0.5 C₄H₄O₄): Verfahren C; Ausbeute 93 %; Schmp. 214 - 219°C (aus Isopropanol).

### Beispiel 4: 6,7-Dimethoxy-3-{2-[4-(2-methoxyphenyl)-1-piperazinyl)ethyl]-4-methyl-2H-1-benzopyran-2-on

Verfahren A; Ausgangsmaterialien: 3-[2-(Methansulfonyloxy)ethyl]-6,7-dimethoxy-4-methyl-2H-1-benzopyran-2-on (Beispiel 28) und 1-(2-Methoxyphenyl)piperazin Hydrochlorid; Ausbeute 14 %; Schrnp. 174 - 175°C (aus Isopropanol).

Fumarat (x 0.5 C₄H₄O₄): Verfahren C; Ausbeute 72 %; Schmp. 227 - 229°C (aus Ethanol).

### Beispiel 5: 6-Ethoxy-7-methoxy-4-methyl-3-[2-(4-phenyl-1-piperidinyl)ethyl]-2H-1-benzopyran-2-on

Verfahren A; Ausgangsmaterialien: 6-Ethoxy-3-[2-(methansulfonyloxy)ethyl]-7-methoxy-4-methyl-2H-1-benzopyran-2-on (Beispiel 29) und 4-Phenylpiperidin; Ausbeute 69 %; Schmp. 195 - 196°C (aus Ethanol).

Fumarat: Verfahren C; Ausbeute 61 %; Schmp. 235 - 238°C (aus Ethanol/Wasser).

### Beispiel 6: 6-Ethoxy-7-methoxy-4-methyl-3-[2-(4-phenyl-1-piperazinyl)ethyl]-2H-1-benzopyran-2-on)

Verfahren B; Ausgangsmaterialien: 6-Ethoxy-3-[2-(methansulfonyloxy)ethyl]-7-methoxy-4-methyl-2H-1-benzopyran-2-on (Beispiel 29) und 1-Phenylpiperazin; Ausbeute 70 %; Schmp. 164 - 165°C (aus Isopropanol).

Fumarat: Verfahren C; Ausbeute 96 %; Schmp. 202 - 204 °C (aus Ethanol).

### Beispiel 7: 6-Ethoxy-7-methoxy-3-{2-[4-(2-methoxyphenyl)-1-piperazinyl]ethyl}-4-methyl-2H-1-benzopyran-2-on

Verfahren A; Ausgangsmaterialien: 6-Ethoxy-3-[2-(methansulfonyloxy)ethyl]-7-methoxy-4-methyl-2H-1-benzopyran-2-on (Beispiel 29) und 1-(2-Methoxyphenyl)piperazin; Ausbeute 54 %; Schmp. 143 - 145°C (aus Ethanol/Chloroform).

Fumarat (x 0.5 C₄H₄O₄): Verfahren C; Ausbeute 83 %; Schmp. 185 - 187°C (aus Ethanol).

### Beispiel 8: 7-Methoxy-3-{2-[4-(2-methoxyphenyl)-1-piperazinyl]ethyl}-4-methyl-6-(1-methylethoxy)-2H-1-benzopyran-2-on

Verfahren A; Ausgangsmaterialien: 3-(2-Chlorethyl)-7-methoxy-4-methyl-6-(1-methylethoxy)-2H-1-benzopyran-2-on (Beispiel 30) und 1-(2-Methoxyphenyl)piperazin Hydrochlorid; Ausbeute 54 %; Schmp. 123 - 125°C (aus Ethanol).

Fumarat: Verfahren C; Ausbeute 91 %; Schmp. 203 - 205°C (aus Ethano/TBME).

### Beispiel 9: 6-(Ethansulfonyloxy)-7-methoxy-3-{2-[4-(2-methoxyphenyl)-1-piperazinyl]-ethyl}-4-methyl-2H-1-benzopyran-2-on

Verfahren A; Ausgangsmaterialien: 3-(2-Chlorethyl)-6-(ethansulfonyloxy)-7-methoxy-4-methyl-2H-1-benzopyran-2-on (Beispiel 31) und 1-(2-Methoxyphenyl)piperazin; Ausbeute 34 % kristallines Rohprodukt.

Alternatives Verfahren: 1.3 g (3.1 mmol) 6-Hydroxy-7-methoxy-3-{2-[4-(2-methoxyphenyl)-1-piperazinyl]ethyl}-4-methyl-2H-1-benzopyran-2-on (Beispiel 2), zwei Äquivalente Natronlauge und 2.5 Äquivalente Ethansulfonsäure werden in Wasser/Ethanol gelöst und 60 h bei Raumtemperatur gerührt. Man nimmt in Chloroform auf, wäscht mit verdünnter Natronlauge und mit Wasser, trocknet über Natriumsulfat und entfernt das Lösungsmittel im Vakuum. Nach Säulenchromatographie über Kieselgel (Laufmittel: Aceton/Petrolether 1/1) erhält man 0.94 g (59 %) beige Kristalle mit Schmp. 148 - 150 °C (aus Ethanol/TBME).

Fumarat (x 0.5 C₄H₄O₄): Verfahren C; Ausbeute 79 %; Schmp. 94 - 95°C (aus Ethanol/TBME).

### Beispiel 10: 7,8-Dimethoxy-3-{2-[4-(2-methoxyphenyl)-1-piperazinyl]ethyl}-4-methyl-2H-1-benzopyran-2-on

Verfahren A; Ausgangsmaterialien: 3-(2-Chlorethyl)-7,8-dimethoxy-4-methyl-2H-1-benzopyran-2-on (Beispiel 32) und 1-(2-Methoxyphenyl)piperazin Hydrochlorid; Ausbeute 6 %; Schmp. 146 - 148°C (aus Isopropanol/TBME).

Fumarat: Verfahren C; Ausbeute 88 %; Schmp. 168 - 170°C (aus Aceton/Petrolether/ TBME).

### Beispiel 11: 7,8-Diethoxy-3-{2-[4-(2-methoxyphenyl)-1-piperazinyl]ethyl}-4-methyl-2H-1-benzopyran-2-on

Verfahren B; Ausgangsmaterialien: 3-(2-Chlorethyl)-7,8-diethoxy-4-methyl-2H-1-benzopyran-2-on (Beispiel 33) und 1-(2-Methoxyphenyl)piperazin; Ausbeute 13 % Rohprodukt. Fumarat: Verfahren C; Ausbeute 89 %; Schmp. 175 - 177°C (aus Aceton).

### Beispiel 12: 5,6,7-Trimethoxy-4-methyl-3-[2-(4-phenyl-1-piperidinyl)ethyl]-2H-1-benzopyran-2-on

Verfahren B; Ausgangsmaterialien: 3-[2-(Methansulfonyloxy)ethyl]-5,6,7-trimethoxy-4-methyl-2H-1-benzopyran-2-on (Beispiel 34) und 4-Phenylpiperidin; Ausbeute 27 %; Schmp. 117 - 118°C (aus TBME/Isopropanol).

Fumarat: Verfahren C; Ausbeute 94 %; Schmp. 187 - 189°C (aus Aceton).

### Beispiel 13: 5,6,7-Trimethoxy-4-methyl-3-[2-(4-phenyl-1-piperazinyl)ethyl]-2H-1-benzopyran-2-on

Verfahren B; Ausgangsmaterialien: 3-[2-(Methansulfonyloxy)ethyl]-5,6,7-trimethoxy-4-methyl-2H-1-benzopyran-2-on (Beispiel 34) und 1-Phenylpiperazin; Ausbeute 49 %; Schmp. 136 - 137°C (aus TBME/Isopropanol).

Fumarat: Verfahren C; Ausbeute 94 %; Schmp. 176 - 178°C (aus Aceton).

### Beispiel 14: 5,7-Dimethoxy-4-methyl-3-[2-(4-phenyl-1-piperidinyl)ethyl]-2H-1-benzopyran-2-on

Verfahren B; Ausgangsmaterialien: 3-[2-(Methansulfonyloxy)ethyl]-5,7-dimethoxy-4-methyl-2H-1-benzopyran-2-on (Beispiel 35) und 4-Phenylpiperidin; Ausbeute 18 % rohes Kristallisat.

Fumarat: Verfahren C; Ausbeute 68 %; Schmp. 220 - 222°C (aus Isopropanol/TBME).

### Beispiel 15: 5,7-Dimethoxy-4-methyl-3-[2-(4-phenyl-1-piperazinyl)ethyl]-2H-1-benzopyran-2-on

Verfahren B; Ausgangsmaterialien: 3-[2-(Methansulfonyloxy)ethyl]-5,7-dimethoxy-4-methyl-2H-1-benzopyran-2-on (Beispiel 35) und 1-Phenylpiperazin; Ausbeute 50 %; Schmp. 112 - 114°C (aus Isopropanol/TBME).

Fumarat (x 0.5 C₄H₄O₄): Verfahren C; Ausbeute 91 %; Schmp. 216 - 218°C (aus Isopropanol/TBME).

### Beispiel 16: 5,7-Dihydroxy-3-{2-[4-(2-methoxyphenyl)-1-piperazinyl]ethyl}-4-methyl-2H-1-benzopyran-2-on

Verfahren B; Ausgangsmaterialien: 3-(2-Chlorethyl)-5,7-dihydroxy-4-methyl-2H-1-benzopyran-2-on (Beispiel 36) und 1-(2-Methoxyphenyl)piperazin; Ausbeute 23 % Rohkristallisat.

Fumarat (x 0.5 C₄H₄O₄): Verfahren C; Ausbeute 80 %; Schmp. 225 - 228°C (aus Ethanol).

### Beispiel 17: 5,7-Dimethoxy-3-{2-[4-(2-methoxyphenyl)-1-piperazinyl]ethyl}-4-methyl-2H-1-benzopyran-2-on

Verfahren B; Ausgangsmaterialien: 3-[2-(Methansulfonyloxy)ethyl]-5,7-dimethoxy-4-methyl-2H-1-benzopyran-2-on (Beispiel 35) und 1-(2-Methoxyphenyl)piperazin; Ausbeute 71 %; Schmp. 140 - 142°C (aus Ethanol).

Fumarat (x 0.5 C₄H₄O₄): Verfahren C; Ausbeute 92 %; Schmp. 185 - 188°C (aus Ethanol).

### Beispiel 18: 5,7-Diethoxy-3-{2-[4-(2-methoxyphenyl)-1-piperazinyl]ethyl}-4-methyl-2H-1-benzopyran-2-on

Verfahren A; Ausgangsmaterialien: 3-(2-Chlorethyl)-5,7-diethoxy-4-methyl-2H-1-benzopyran-2-on (Beispiel 37) und 1-(2-Methoxyphenyl)piperazin; Ausbeute 52 % öliges Rohprodukt.

Fumarat (x 0.5 C₄H₄O₄): Verfahren C; Ausbeute 26 %; Schmp. 210 - 213°C (aus Ethanol/Wasser).

### Beispiel 19: 3-{2-[4-(2-Methoxyphenyl)-1-piperazinyl]ethyl}-4-methyl-5,7-dipropoxy-2H-1-benzopyran-2-on

Verfahren B; Ausgangsmaterialien: 3-(2-Chlorethyl)-4-methyl-5,7-dipropoxy-2H-1-benzopyran-2-on (Beispiel 38) und 1-(2-Methoxyphenyl)piperazin; Ausbeute 47 % Rohprodukt. Fumarat: Verfahren C; Ausbeute 49 %; Schmp. 178 - 180°C (aus Ethanol).

### Beispiel 20: 3-{2-[4-(2-Ethoxyphenyl)-1-piperazinyl]ethyl}-5,7-dimethoxy-4-methyl-2H-1-benzopyran-2-on

Verfahren B; Ausgangsmaterialien: 3-(2-Chlorethyl)-5,7-dimethoxy-4-methyl-2H-1-benzopyran-2-on (Beispiel 39) und 1-(2-Ethoxyphenyl)piperazin; Ausbeute 31 % Rohprodukt. Fumarat: Verfahren C; Ausbeute 87 %; Schmp. 205 - 206°C (Zers.; aus Aceton).

### Beispiel 21: 5,7-Dimethoxy-4-methyl-3-{2-[4-(2-methylphenyl)-1-piperazinyl]ethyl}-2H-1-benzopyran-2-on

Verfahren A; Ausgangsmaterialien: 3-(2-Chlorethyl)-5,7-dimethoxy-4-methyl-2H-1-benzopyran-2-on (Beispiel 39) und 1-(2-Methylphenyl)piperazin; Ausbeute 14 % öliges Rohprodukt.

Fumarat (x 0.5 C₄H₄O₄): Verfahren C; Ausbeute 57 %; Schmp. 218 - 220°C (aus Ethanol).

### Beispiel 22: 3-{2-[4-(2-Fluorphenyl)-1-piperazinyl]ethyl}-5,7-dimethoxy-4-methyl-2H-1-benzopyran-2-on

Verfahren B; Ausgangsmaterialien: 3-(2-Chlorethyl)-5,7-dimethoxy-4-methyl-2H-1-benzopyran-2-on (Beispiel 39) und 1-(2-Fluorphenyl)piperazin; Ausbeute 42 %; Schmp. 152-153°C (aus Ethanol).

Fumarat: Verfahren C; Ausbeute 93 %; Schmp. 237 - 238°C (Zers.; aus Aceton).

### Beispiel 23: 5,7-Dimethoxy-4-methyl-3-{2-[4-(3-trifluormethylphenyl)-1-piperazinyl]ethyl}-2H-1-benzopyran-2-on

Verfahren B; Ausgangsmaterialien: 3-(2-Chlorethyl)-5,7-dimethoxy-4-methyl-2H-1-benzopyran-2-on (Beispiel 39) und 1-(3-Trifluormethylphenyl)piperazin; Ausbeute 47 %; Schmp. 138 - 139°C (aus Isopropanol).

Fumarat: Verfahren C; Ausbeute 88 %; Schmp. 224 - 226°C (aus Isopropanol/TBME).

### Beispiel 24: 3-[2-(4-Hydroxy-4-phenyl-1-piperidinyl)ethyl]-5,7-dimethoxy-4-methyl-2H-1-benzopyran-2-on

Verfahren B; Ausgangsmaterialien: 3-(2-Chlorethyl)-5,7-dimethoxy-4-methyl-2H-1-benzopyran-2-on (Beispiel 39) und 4-Hydroxy-4-phenylpiperidin; Ausbeute 41 %; Schmp. 177.5-179°C (aus Ethylacetat / Ethanol).

Fumarat (x 0.5 C₄H₄O₄): Verfahren C; Ausbeute 60 %; Schmp. 218 - 220°C (aus Aceton / Wasser).

### Beispiel 25: 3-{2-[4-(2-Hydroxyphenyl)-1-piperazinyl]ethyl}-5,7-dimethoxy-4-methyl-2H-1-benzopyran-2-on

Verfahren B; Ausgangsmaterialien: 3-(2-Chlorethyl)-5,7-dimethoxy-4-methyl-2H-1-benzopyran-2-on (Beispiel 39) und 1-(2-Hydroxyphenyl)piperazin; Ausbeute 22 %; Schmp. 164-165°C (aus Ethanol / TBME).

### II. Beispiele 26 bis 39 für Zwischenprodukte der allgemeinen Formel II

Zur Herstellung der in den nachfolgenden Beispielen 26 bis 39 näher beschriebenen Verbindungen wurden folgende Verfahren benutzt:

Verfahren D: Zur Lösung des entsprechend mit R¹ bis R⁴ substituierten Alkohols der allgemeinen Formel IV und von 1.5 Äquivalenten Triethylamin in Chloroform tropft man bei 0 bis 25°C eine Lösung von 1.5 Äquivalenten Methansulfonsäurechlorid in Chloroform und rührt wenige Minuten bis 16 h bei 0 bis 25°C. Falls das Produkt ausfällt, wird es abgesaugt und gegebenenfalls mit Wasser gewaschen. Die Chloroformlösung wird mit Wasser gewaschen, mit Na₂SO₄ getrocknet, einrotiert, gegebenenfalls chromatographiert und umkristallisiert.

Verfahren E: Der entsprechend mit R¹ bis R⁴ substituierte Alkohol der allgemeinen Formel IV wird unter Rühren tropfenweise mit 2 bis 4 Äquivalenten Thionylchlorid versetzt. Man rührt 1 bis 20 h bei Raumtemperatur und tropft dann vorsichtig Wasser zu. Das Produkt fällt aus, wird abgesaugt, gegebenenfalls chromatographiert und umkristallisiert.

### Beispiel 26: 3[2-(Methansulfonyloxy)ethyl]-7-methoxy-4-methyl-2H-1-benzopyran-2-on

Verfahren D; Ausgangsmaterial: 3-(2-Hydroxyethyl)-7-methoxy-4-methyl-2H-1-benzopyran-2-on (Beispiel 40); Ausbeute 82 %; Schmp. 174 - 175°C (aus Aceton).

### Beispiel 27: 3-(2-Chlorethyl)-6-hydroxy-7-methoxy-4-methyl-2H-1-benzopyran-2-on

Verfahren E; Ausgangsmaterial: 6-Hydroxy-3-(2-hydroxyethyl)-7-methoxy-4-methyl-2H-1-benzopyran-2-on (Beispiel 41); Ausbeute 98 %; Schmp. 230 - 235°C (aus Ethylacetat/Ethanol).

### Beispiel 28: 3-[2-(Methansulfonyloxy)ethyl]-6,7-dimethoxy-4-methyl-2H-1-benzopyran-2-on

Verfahren D; Ausgangsmaterial: 3-(2-Hydroxyethyl)-6,7-dimethoxy-4-methyl-2H-1-benzopyran-2-on (Beispiel 42); Ausbeute 72 %; Schmp. 188 - 191°C (ausgerührt mit TBME).

### Beispiel 29: 6-Ethoxy-3-[2-(methansulfonyloxy)ethyl]-7-methoxy-4-methyl-2H-1-benzopyran-2-on

Verfahren D; Ausgangsmaterial: 6-Ethoxy-3-(2-hydroxyethyl)-7-methoxy-4-methyl-2H-1-benzopyran-2-on (Beispiel 43); Ausbeute 94 %; Schmp. 153 - 154°C (ausgerührt mit TBME).

### Beispiel 30: 3-(2-Chlorethyl)-7-methoxy-4-methyl-6-(1-methylethoxy)-2H-1-benzopyran-2-on

Verfahren E; Ausgangsmaterial: 3-(2-Hydroxyethyl)-7-methoxy-4-methyl-6-(1-methylethoxy)-2H-1-benzopyran-2-on (Beispiel 44); Ausbeute 99 %; Schmp. 125 - 127°C (aus Ethanol).

### Beispiel 31: 3-(2-Chlorethyl)-6-(ethansulfonyloxy)-7-methoxy-4-methyl-2H-1-benzopyran-2-on

Zu 7.0 g (26.1 mmol) 3-(2-Chlorethyl)-6-hydroxy-7-methoxy-4-methyl-2H-1-benzopyran-2-on (Beispiel 27) in 50 ml Wasser wird 1 Äquivalent Natronlauge gegeben. Man rührt 1 h bei Raumtemperatur und tropft dann 4.0 g (31.1 mmol) Ethansulfonsäurechlorid zu. Man rührt 20 h bei Raumtemperatur, tropft weitere 4.0 g (31.1 mmol) Ethansulfonsäurechlorid zu und rührt weitere 5 h. Das Produkt wird abgesaugt und mit Wasser gewaschen. Ausbeute 5.5 g (59 %); Schmp. 154 - 155°C (aus Ethanol/Petrolether).

### Beispiel 32: 3-(2-Chlorethyl)-7,8-dimethoxy-4-methyl-2H-1-benzopyran-2-on

Verfahren E; Ausgangsmaterial: 3-(2-Hydroxyethyl)-7,8-dimethoxy-4-methyl-2H-1-benzopyran-2-on (Beispiel 45); Ausbeute 88 %; Schmp. 137 - 139°C (aus Isopropanol).

### Beispiel 33: 3 (2-Chlorethyl)-7,8-diethoxy-4-methyl-2H-1benzopyran-2-on

Verfahren E; Ausgangsmaterial: 3-(2-Hydroxyethyl)-7,8-diethoxy-4-methyl-2H-1-benzopyran-2-on (Beispiel 46); Ausbeute 85 %; Schmp. 89 - 90°C (aus Isopropanol).

### Beispiel 34: 3-[2-(Methansulfonyloxy)ethyl]-5,6,7-trimethoxy-4-methyl-2H-1-benzopyran-2-on

Verfahren D; Ausgangsmaterial: 3-(2-Hydroxyethyl)-5,6,7-trimethoxy-4-methyl-2H-1-benzopyran-2-on (Beispiel 47); Ausbeute 84 %; Schmp. 114 - 115°C (aus Ethanol).

### Beispiel 35: 3-[2-(Methansulfonyloxy)ethyl]-5,7-dimethoxy-4-methyl-2H-1-benzopyran-2-on

Verfahren D; Ausgangsmaterial: 3-(2-Hydroxyethyl)-5,7-dimethoxy-4-methyl-2H-1-benzopyran-2-on (Beispiel 48); Ausbeute 92 %; Schmp. 170 - 172°C (aus Ethanol/Chloroform).

### Beispiel 36: 3-(2-Chlorethyl)-5,7-dihydroxy-4-methyl-2H-1-benzopyran-2-on

Verfahren E; Ausgangsmaterial: 5,7-Dihydroxy-3-(2-hydroxyethyl)-4-methyl-2H-1-benzopyran-2-on (Beispiel 49); Ausbeute 53 %; Schmp. 218 - 220°C (x 1.0 Aceton; aus Aceton / TBME / Petrolether).

### Beispiel 37: 3 -(2-Chlorethyl)-5,7-diethoxy-4-methyl-2H-1-benzopyran-2-on

Verfahren E; Ausgangsmaterial: 5,7-Diethoxy-3-(2-hydroxyethyl)-4-methyl-2H-l-benzopyran-2-on (Beispiel 50); Ausbeute 83 %; Schmp. 123 - 126°C (aus Chloroform/Ethanol).

### Beispiel 38: 5,7-Bis(propyloxy)-3-(2-chlorethyl)-4-methyl-2H-1-benzopyran-2-on

Verfahren E; Ausgangsmaterial: 5,7-Bis(propyloxy)-3-(2-hydroxyethyl)-4-methyl-2H-1-benzopyran-2-on (Beispiel 51); Ausbeute 88 %; Schmp. 99 - 101°C (aus Isopropanol).

### Beispiel 39: 3-(2-Chlorethyl)-5,7-dimethoxy-4-methyl-2H-1-benzopyran-2-on

Verfahren E; Ausgangsmaterial: 3-(2-Hydroxyethyl)-5,7-dimethoxy-4-methyl-2H-1-benzopyran-2-on (Beispiel 48); Ausbeute 73 %; Schmp. 129 - 130°C (aus Isopropanol).

### III. Beispiele 40 bis 52 für Zwischenprodukte der allgemeinen Formel IV

Zur Herstellung der in den nachfolgenden Beispielen 40 bis 52 näher beschriebenen Verbindungen wurden folgende Verfahren benutzt:

Verfahren F: Das entsprechend mit R¹ bis R³ substituierte Phenol der allgemeinen Formel V wird in etwa der vier- bis sechsfachen Gewichtsmenge 75-prozentiger wässriger Schwefelsäure suspendiert. Bei 0 bis 25°C tropft man dazu unter Rühren ein Äquivalent 2-Acetyl-γ-butyrolacton, rührt noch 1 bis 18 h bei Raumtemperatur und verdünnt dann mit Eis/Wasser. Fällt das Produkt dabei als fester Niederschlag an, so wird gegebenenfalls noch einige Stunden bei Raumtemperatur gerührt, abgesaugt, mit Wasser gewaschen, im Vakuum getrocknet und umkristallisiert. Andernfalls wird mit Chloroform extrahiert, die organische Phase mit Wasser gewaschen, mit Na₂SO₄ getrocknet, einrotiert, gegebenenfalls chromatographiert und umkristallisiert.

### Beispiel 40: 3-(2-Hydroxyethyl)-7-methoxy-4-methyl-2H-1-benzopyran-2-on

Verfahren F; Ausgangsmaterial: 3-Methoxyphenol; Ausbeute 16 %; Schmp. 141 - 142°C (aus Isopropanol/TBME).

### Beispiel 41: 6-Hydroxy-3-(2-hydroxyethyl)-7-methoxy-4-methyl-2H-1-benzopyran-2-on

Verfahren F; Ausgangsmaterial: Methoxyhydrochinon; Ausbeute 54 %; Schmp. 229-235°C (aus Methanol).

### Beispiel 42: 3-(2-Hydroxyethyl)-6,7-dimethoxy-4-methyl-2H-1-benzopyran-2-on

40.0 g (160 mmol) 6-Hydroxy-3-(2-hydroxyethyl)-7-methoxy-4-methyl-2H-1-benzopyran-2-on (Beispiel 41), 45.4 g (320 mmol) Methyliodid und 66.3 g (480 mmol) Kaliumcarbonat in 500 ml DMF werden 22 h bei 60 bis 80°C gerührt. Man evaporiert, extrahiert mit Chloroform, wäscht die organische Phase mit verd. Natronlauge und mit Wasser, trocknet mit Na₂SO₄ und evaporiert erneut. Ausbeute 31.6 g (75 %); Schmp. 184 - 187°C (aus Isopropanol).

### Beispiel 43: 6-Ethoxy-3-(2-hydroxyethyl)-7-methoxy-4-methyl-2H-1-benzopyran-2-on

Man befreit 4.72 g (118 mmol) Natriumhydrid (60 % in Weißöl) vom Öl, suspendiert es in 100 ml DMF und tropft bei Raumtemperatur eine Lösung von 26.8 g (107 mmol) 6-Hydroxy-3 -(2-hydroxyethyl)-7-methoxy-4-methyl-2H-1-benzopyran-2-on (Beispiel 41) in 500 ml DMF zu. Nach 30 min gibt man 25.1 g (161 mmol) Ethyliodid zu und rührt 14 h bei 80 bis 90°C. Man evaporiert, extrahiert mit Chloroform, wäscht die organische Phase mit verd. Natronlauge und mit Wasser, trocknet mit Na₂SO₄ und evaporiert erneut. Ausbeute 25.1 g (84 %); Schmp. 156 - 157°C (aus Isopropanol).

### Beispiel 44: 3-(2-Hydroxyethyl)-7-methoxy-4-methyl-6-(1-methylethoxy)-2H-1-benzopyran-2-on

Zu 11.8 g (295 mmol) Natriumhydrid (60 % in Weißöl) in 300 ml DMF werden 67 g (268 mmol) 6-Hydroxy-3-(2-hydroxyethyl)-7-methoxy-4-methyl-2H-1-benzopyran-2-on (Beispiel 41) unter Eisbadkühlung vorsichtig zugegeben. Man rührt 1 h bei Raumtemperatur, tropft dann 49.5 g (402 mmol) 2-Brompropan zu und rührt 24 h bei 60°C unter N₂. Nach Zugabe weiterer 3.33 g (83 mmol) Natriumhydrid und 52.4 g (426 mmol) 2-Brompropan wird weitere 24 h bei 60°C gerührt. Man evaporiert, extrahiert mit Ethylacetat, wäscht die organische Phase mit Wasser, trocknet mit Na₂SO₄ und evaporiert erneut. Man reinigt säulenchromatographisch über Kieselgel 60 (Eluens: Ethylacetat). Ausbeute 53.3 g (68 %); Schmp. 110 - 112°C (aus Aceton/PE).

### Beispiel 45: 3-(2-Hydroxyethyl)-7,8-dimethoxy-4-methyl-2H-1-benzopyran-2-on

18.4 g (279 mmol) Kaliumhydroxid (85 %) und 30.0 g (127 mmol) 7,8-Dihydroxy-3-(2-hydroxyethyl)-4-methyl-2H-1-benzopyran-2-on (Beispiel 52) werden in 700 ml Ethanol 30 min bei Raumtemperatur gerührt. Man gibt 39.7 g (280 mmol) Methyliodid zu und rührt 32 h bei 60°C. Nach Zugabe weiterer 2.11 g (32 mmol) Kaliumhydroxid und 4.54 g (32 mmol) Methyliodid wird weitere 8 h bei 60°C gerührt. Man evaporiert, extrahiert mit Chloroform, wäscht die organische Phase mit Wasser, trocknet mit Na₂SO₄ und evaporiert erneut. Ausbeute 16.15 g (48 %); Schmp. 113 - 114°C (aus TBME/Isopropanol).

### Beispiel 46: 7,8-Diethoxy-3-(2-hydroxyethyl)-4-methyl-2H-1-benzopyran-2-on

30.0 g (127 mmol) 7,8-Dihydroxy-3-(2-hydroxyethyl)-4-methyl-2H-1-benzopyran-2-on (Beispiel 52), 52.7 g (381 mmol) Kaliumcarbonat und 43.6 g (280 mmol) Ethyliodid werden in 800 ml Ethanol 50 h unter Rückfluß gerührt. Man evaporiert, extrahiert mit Chloroform, wäscht die organische Phase mit Wasser, trocknet mit Na₂SO₄ und evaporiert erneut. Ausbeute 21.0 g (57 %); Schmp. 130 - 131°C (aus Isopropanol).

### Beispiel 47: 3-(2-Hydroxyethyl)-5,6,7-trimethoxy-4-methyl-2H-1-benzopyran-2-on

Verfahren F; Ausgangsmaterial: 3,4,5-Trimethoxyphenol; Ausbeute 29 %; Schmp. 95-97°C (aus Petrolether/TBME).

### Beispiel 48: 3-(2-Hydroxyethyl)-5,7-dimethoxy-4-methyl-2H-1-benzopyran-2-on

Verfahren F; Ausgangsmaterial: 3,5-Dimethoxyphenol; Ausbeute 72 %; Schmp. 128-130°C (aus Isopropanol/TBME).

Alternatives Verfahren: 100.0 g (423 mmol) 5,7-Dihydroxy-3-(2-hydroxyethyl)-4-methyl-2H-1-benzopyran-2-on (Beispiel 49), 175.5 g (1270 mmol) Kaliumcarbonat und 150.2 g (1058 mmol) Mehyliodid werden in 1800 ml Ethanol 4 h unter Rückfluß gerührt. Man filtriert, evaporiert, extrahiert mit Chloroform, wäscht die organische Phase mit Wasser, trocknet mit Na₂SO₄ und evaporiert erneut. Ausbeute 49.3 g (44 %); Schmp. 148 - 150°C (aus Isopropanol / TBME).

### Beispiel 49: 5,7-Dihydroxy-3-(2-hydroxyethyl)-4-methyl-2H-1-benzopyran-2-on

Verfahren F; Ausgangsmaterial: Phloroglucin-Dihydrat; Ausbeute 88 %; Schmp. 243-245°C (aus Ethanol/Wasser).

### Beispiel 50: 5,7-Diethoxy-3-(2-hydroxyethyl)-4-methyl-2H-1-benzopyran-2-on

33.0 g (140 mmol) 5,7-Dihydroxy-3-(2-hydroxyethyl)-4-methyl-2H-1-benzopyran-2-on (Beispiel 49), 56.0 g (560 mmol) Kaliumhydrogencarbonat und 76.4 g (490 mmol) Ethyliodid werden in 500 ml DMF 55 h bei 80°C unter N₂ gerührt. Man evaporiert, extrahiert mit Chloroform, wäscht die organische Phase mit verd. Natronlauge und mit Wasser, trocknet mit Na₂SO₄ und evaporiert erneut. Man reinigt säulenchromatographisch über Kieselgel 60 (Eluens: Ethylacetat/Aceton). Ausbeute 19.5 g (48 %); Schmp. 117-119°C (aus Aceton/Petrolether).

### Beispiel 51: 5,7-Bis(propyloxy)-3-(2-hydroxyethyl)4-methyl-2H-1-benzopyran-2-on

30.0 g (127 mmol) 5,7-Dihydroxy-3-(2-hydroxyethyl)4-methyl-2H-1-benzopyran-2-on (Beispiel 49), 50.8 g (507 mmol) Kaliumhydrogencarbonat, 54.7 g (445 mmol) 1-Brompropan und 5.0 g Kaliumiodid werden in 500 ml DMF 43 h bei 80°C gerührt. Man evaporiert, extrahiert mit Chloroform, wäscht die organische Phase mit Wasser, trocknet mit Na₂SO₄ und evaporiert erneut. Man reinigt säulenchromatographisch über Kieselgel 60 (Eluens: Ethylacetat/Petrolether 9/1). Ausbeute 28.8 g (71 %); Schmp. 106 - 108°C (aus Petrolether/Aceton).

### Beispiel 52: 7,8-Dihydroxy-3-(2-hydroxyethyl)-4-methyl-2H-1-benzopyran-2-on

Verfahren F; Ausgangsmaterial: Pyrogallol; Ausbeute 66 %; Schmp. 221 - 222°C (aus Ethanol/Wasser).

### Pharmakologische Untersuchungen

Zur Ermittlung der neuroprotektiven / antikonvulsiven Aktivität der erfindungsgemäßen Verbindungen der allgemeinen Formel I wurde folgende Methode verwendet:

Zur Charakterisierung neuroprotektiver Wirksamkeit wurde männlichen NMRI-Mäusen mit einem Körpergewicht von 20 - 25 g NMDA in einer Menge von 25 mg/kg/10 ml intravenös appliziert. Als Folge dieser Applikation erleiden die Tiere klonische und teilweise auch tonische Konvulsionen, die zum Tode führen. Als Kriterium für die Wirksamkeit der untersuchten Verbindungen dient die Verhinderung des Todes.

Alle Versuchstiere hatten vor den Experimenten freien Zugang zu Futter und Wasser. Die Test- und Referenzsubstanzen wurden als Suspension in 0.2 % Agar oder Wasser, z. T. unter Zuhilfenahme von Lösungsvermittlern wie z. B. PEG-400, peroral durch Schlundsonde verabreicht. Kontrolltiere erhielten gleiche Volumina der Lösungsmittel gegebenenfalls unter Zusatz von Lösungsvermittlern appliziert. Eine Stunde nach Substanzapplikation wurde NMDA intravenös appliziert und das Auftreten von Konvulsionen beobachtet.

In Tabelle 1 sind die Ergebnisse der durchgeführten Tests (NMDA 25 mg i.v.) bei Dosen von 5 mg der erfindungsgemäßen Verbindungen pro kg Körpergewicht im Vergleich zu bekannten NMDA-Antagonisten aufgeführt. Als %-Wirkung ist der prozentuale Anteil der überlebenden Tiere angegeben.

In der Tabelle 1 sind außerdem als ED-50-Werte diejenigen Dosen der erfindungsgemäßen Substanzen und anderer NMDA-Antagonisten aufgeführt, die im NMDA-i.v.-Test (25 mg/kg) eine Stunde nach Applikation 50 % der Tiere vor dem NMDA-induzierten Tod schützen.

Die Bestimmung der ED50-Werte erfolgte nach [Lichtfield und Wilcoxon, J. Pharmacol. exp. Therapeut. 96, 99 (1949)] jeweils mit 4 - 5 Tiergruppen zu 10 Tieren je Dosisstufe.

Während der gesamten Versuchsdauer wurden die Tiere auf Anzeichen substanzbedingter Verhaltensänderungen und Neurotoxizität beobachtet. Bei sämtlichen erfindungsgemäßen Verbindungen wurden in den verwendeten Dosierungen keinerlei Anzeichen einer toxischen Eigenwirkung beobachtet.

**Tabelle 1 :**

| Ergebnisse des NMDA-Tests (25 mg/kg iv.) und die ED-50 Werte | | | | |
|---|---|---|---|---|
| Substanz gemäß Beispiel Nr. | Dosis (mg/kg po.) 1 Std. vor Test | Anzahl Tiere im Versuch | Schutzwirkung (%) | ED-50 (mg/kg po.) |
| 1 | 5 | 8 | 87.5 | 0.8 |
| 2 | 5 | 8 | 100 | 0.2 |
| 3 | 5 | 8 | 50 | |
| 4 | 5 | 8 | 100 | 0.4 |
| 5 | 5 | 8 | 75 | 4.1 |
| 6 | 5 | 8 | 87.5 | 2.0 |
| 7 | 5 | 8 | 100 | 0.4 |
| 8 | 5 | 8 | 100 | 0.7 |
| 9 | 5 | 8 | 100 | 0.6 |
| 10 | 5 | 8 | 100 | 0.4 |
| 11 | 5 | 8 | 100 | |
| 12 | 5 | 8 | 75 | 3.4 |
| 13 | 5 | 8 | 62.5 | |
| 14 | 5 | 8 | 87.5 | 2.2 |
| 15 | 5 | 8 | 87.5 | 1.0 |
| 16 | 5 | 8 | 100 | 0.2 |
| 17 | 5 | 8 | 100 | 0.2 |
| 18 | 5 | 8 | 100 | 0.6 |
| 19 | 5 | 8 | 100 | |
| 20 | 5 | 8 | 100 | |
| 21 | 5 | 8 | 87.5 | |
| 22 | 5 | 8 | 100 | |
| 23 | 5 | 8 | 25 | |
| 24 | 5 | 8 | 25 | |
| 25 | 5 | 8 | 87.5 | |
| | | | | |
| Flunarizin | 50 | 8 | 100 | 22.8 |
| Nimodipin | 20 | 8 | 87.5 | 18.5 |
| Verapamil | 20 | 8 | 87.5 | 17.3 |
| Dextromethorphan | 50 | 8 | 50 | 66.7 |
| Ketamin | 20 | 8 | 0 | |

### Beispiele für die Herstellung pharmazeutischer Zubereitungen der erfindungsgemäßen Substanzen:

### A. Tabletten:

Zur Herstellung von Tabletten, die je nach gewünschter Wirkungsstärke 5 bis 250 mg Wirkstoff enthalten, benötigt man

| | |
|---|---|
| erfindungsgemäße Substanz | 200 bis 5 000 g |
| Zellulosepulver | 2 000 g |
| Maisstärke | 1 200 g |
| kolloide Kieselsäure | 80 g |
| Magnesiumstearat | 20 g |
| Milchzucker | ad 10 000 g |

Der Wirkstoff wird gegebenenfalls gemahlen, mit den hilfsstoffen homogen vermischt und in der üblichen Weise zu Tabletten von je 250 mg Gewicht und 9 mm Durchmesser verpreßt. Bei Dosierungen über 125 mg preßt man Tabletten von je 500 mg Gewicht und 11 mm Durchmesser. Falls gewünscht, werden die Tabletten mit einem Filmüberzug versehen.

### B. Kapseln:

Zur Herstellung von Kapseln, die je nach gewünschter Wirkungsstärke 5 bis 250 mg Wirkstoff enthalten, benötigt man

| | |
|---|---|
| erfindungsgemäße Substanz | 500 bis 12 500 g |
| Maisstärke | 2 000 g |
| kolloide Kieselsäure | 300 g |
| Magnesiumstearat | 50 g |
| Zellulosepulver | ad 20 000 g |

Die feingepulverten Stoffe werden homogen gemischt und in Hartgelatinekapseln der Größe 2 in der Menge von 200 mg pro Kapsel, oder bei Dosierungen über 125 mg in Hartgelatinekapseln der Größe 0 in der Menge von 400 mg pro Kapsel abgefüllt.

## Patentansprüche

1. 2H-1-Benzopyran-2-one der allgemeinen Formel I, 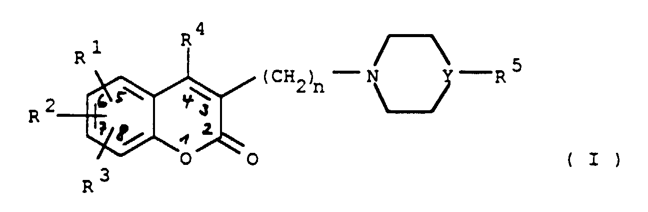 worin
R¹ einen Hydroxyrest, einen Alkoxyrest mit 1 bis 5 C-Atomen, einen Cycloalkoxyrest mit 4 bis 6 C-Atomen oder einen Alkyl- oder Arylsulfonyloxyrest R⁶-SO₂O-,
R² und R³ unabhängig voneinander Wasserstoffatome, Hydroxyreste, Alkoxyreste mit 1 bis 4 C-Atomen oder Cycloalkoxyreste mit 4 bis 6 C-Atomen,
R⁴ ein Wasserstoffatom, eine Alkylgruppe mit 1 bis 4 C-Atomen oder einen Phenylrest,
Y ein Stickstoffatom, eine CH-Gruppe oder eine COH-Gruppe,
R⁵ einen Phenyl-, Naphthyl-, Pyridinyl- oder Pyrimidinyl-Rest, der gegebenenfalls mit jeweils ein oder zwei C₁-C₅-Alkylgruppen, mit jeweils ein oder zwei Halogenatomen, mit Halogen und gleichzeitig C₁-C₅-Alkyl, mit Perfluoralkyl mit 1 bis 3 C-Atomen, C₁-C₅-Alkoxy, Hydroxy, Methylendioxy oder Nitro substituiert ist,
R⁶ einen Alkylrest mit 1 bis 5 C-Atomen, einen Cycloalkylrest mit 4 bis 6 C-Atomen oder einen Phenylrest, der gegebenenfalls mit jeweils ein oder zwei C₁-C₅-Alkylgruppen, mit jeweils ein oder zwei Halogenatomen oder mit Perfluoralkyl mit 1 bis 3 C-Atomen substituiert ist, und
n eine ganze Zahl von 1 bis 4
bedeuten, sowie deren Additionsverbindungen mit physiologisch verträglichen Säuren, jedoch mit Ausnahme von 7,8-Dimethoxy-4-methyl-3-[(4-phenyl-1-piperazinyl)methyl]-2H-1-benzopyran-2-on.

2. Verbindungen der Formel I nach Anspruch 1, worin
R¹ einen Hydroxy-, Methoxy-, Ethoxy-, Propyloxy- oder Ethansulfonyloxyrest,
R² und R³ unabhängig voneinander Wasserstoffatome, Hydroxy- oder Alkoxyreste mit 1 bis 3 C-Atomen,
R⁴ eine Methylgruppe,
Y ein Stickstoffatom, eine CH-Gruppe oder eine COH-Gruppe,
R⁵ einen ggf. mit Hydroxy, Methoxy, Ethoxy, Methyl, Fluor oder Trifluormethyl substituierten Phenylrest und
n=2
bedeuten, sowie deren Additionsverbindungen mit physiologisch verträglichen Säuren.

3. Verfahren zur Herstellung der Verbindungen gemäß Anspruch 1 oder 2, dadurch gekennzeichnet, daß eine Verbindung der allgemeinen Formel II 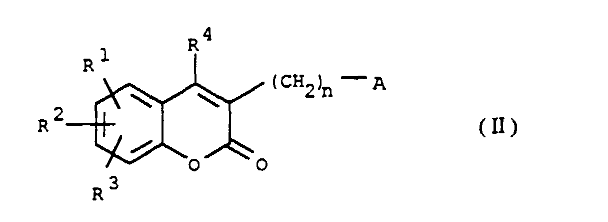 worin R¹, R², R³, R⁴ und n die in Anspruch 1 angegebenen Bedeutungen haben und A eine Abgangsgruppe ist, die ausgewählt ist aus der Gruppe Chlor, Brom, Iod, Alkylsulfonyloxy, Trifluormethansulfonyloxy, gegebenenfalls mit Alkyl, Nitro oder Halogen substituiertes Phenylsulfonyloxy, mit einer Verbindung der allgemeinen Formel III 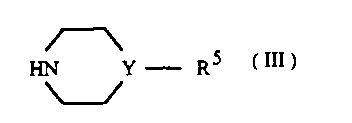 worin Y und R⁵ die in Anspruch 1 angegebenen Bedeutungen haben, unter Eliminierung der Säure HA umgesetzt und das entstandene Reaktionsprodukt der allgemeinen Formel I gegebenenfalls in eine physiologisch verträgliche Säureadditionsverbindung übergeführt wird.

4. Verfahren nach Anspruch 3, dadurch gekennzeichnet, daß die Umsetzung jeweils in Gegenwart einer Base durchgeführt wird, die ausgewählt ist aus der Gruppe der Alkali- und Erdalkalicarbonate, -hydrogencarbonate, -hydride, -alkoholate, -hydroxide und tertiären Amine.

5. Verfahren nach Anspruch 3 oder 4, dadurch gekennzeichnet, daß die Umsetzung in Gegenwart eines gegenüber den Reaktanden inerten Lösungsmittels aus der Gruppe der Alkanole, aromatischen Lösungsmittel, aliphatischen und cycloaliphatischen Ether, Carbonsäuredialkylamide, Tetraalkylharnstoffe, Ketone und Sulfoxide durchgeführt wird.

6. Verfahren nach einem der Ansprüche 3 bis 5, dadurch gekennzeichnet, daß die Umsetzung in Gegenwart eines Katalysators aus der Gruppe der Alkali- und Erdalkaliiodide durchgeführt wird.

7. Verfahren nach einem der Ansprüche 3 bis 6, dadurch gekennzeichnet, daß die Umsetzung bei einer Temperatur zwischen Raumtemperatur und 130°C, vorzugsweise bei einer Temperatur von weniger als 100°C, und bei einem Druck zwischen Normaldruck und 10⁷ Pa durchgefiihrt wird.

8. Verfahren nach einem der Ansprüche 3 bis 7, dadurch gekennzeichnet, daß die Umsetzung unter einer Schutzgasatmosphäre wie N₂ oder Ar durchgeführt wird.

9. Pharmazeutische Zusammensetzung, enthaltend mindestens eine Verbindung gemäß einem der Ansprüche 1 oder 2, gegebenenfalls zusammen mit üblichen Hilfs- und Zusatzstoffen.

10. Verwendung der Verbindungen gemäß einem der Ansprüche 1 oder 2 zur Herstellung eines Arzneimittels mit neuroprotektiver, antikonvulsiver und/oder antiepileptischer Wirksamkeit.

11. Verwendung der Verbindungen nach Anspruch 10 zur Herstellung eines Arzneimittels mit zusätzlich antidepressiver, nootropischer, antipsychotischer und/oder anxiolytischer Wirksamkeit.

## Claims

1. 2H-1-benzopyran-2-ones of the general formula I, 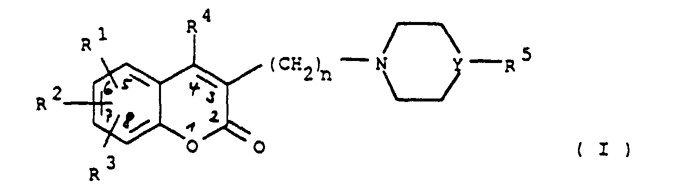 in which
R¹ is a hydroxy radical, an alkoxy radical with 1 to 5 C atoms, a cycloalkoxy radical with 4 to 6 C atoms, or an alkyl- or arylsulphonyloxy radical R⁶-SO₂O-,
R² and R³ are, independently of one another, hydrogen atoms, hydroxy radicals, alkoxy radicals with 1 to 4 C atoms, or cycloalkoxy radicals with 4 to 6 C atoms,
R⁴ is a hydrogen atom, an alkyl group with 1 to 4 C atoms, or a phenyl radical,
Y is a nitrogen atom, a CH group or a COH group,
R⁵ is a phenyl, naphthyl, pyridinyl or pyrimidinyl radical, which is optionally substituted with in each case one or two C₁-C₅ alkyl groups, with in each case one or two halogen atoms, with halogen and simultaneously C₁-C₅ alkyl, with perfluoroalkyl with 1 to 3 C atoms, C₁-C₅ alkoxy, hydroxy, methylenedioxy or nitro,
R⁶ is an alkyl radical with 1 to 5 C atoms, a cycloalkyl radical with 4 to 6 C atoms or a phenyl radical, which is optionally substituted with in each case one or two C₁-C₅ alkyl groups, with in each case one or two halogen atoms or with perfluoroalkyl with 1 to 3 C atoms, and
n is an integer from 1 to 4,
as well as their addition compounds with physiologically compatible acids, with the exception however of 7,8-dimethoxy-4-methyl-3-[(4-phenyl-1-piperazinyl)methyl]-2H-1-benzopyran-2-one.

2. Compounds of the formula I according to Claim 1, wherein
R¹ is a hydroxy, methoxy, ethoxy, propyloxy or ethane sulphonyloxy radical,
R² and R³ are, independently of one another, hydrogen atoms, hydroxy or alkoxy radicals with 1 to 3 C atoms,
R⁴ is a methyl group,
Y is a nitrogen atom, a CH group or a COH group,
R⁵ is a phenyl radical optionally substituted with hydroxy, methoxy, ethoxy, methyl, fluoro or trifluoromethyl, and
n = 2
as well as their addition compounds with physiologically compatible acids.

3. Process for preparing the compounds according to Claim 1 or 2, characterised in that a compound of the general formula II 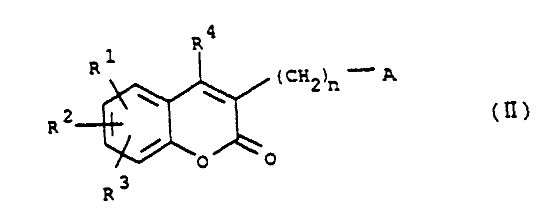 wherein R¹, R², R³, R⁴ and n have the meanings given in Claim 1 and A is a leaving group selected from chlorine, bromine, iodine, alkylsulphonyloxy, trifluoromethanesulphonyloxy, phenylsulphonyloxy optionally substituted with alkyl, nitro or halogen, is reacted with a compound of the general formula III 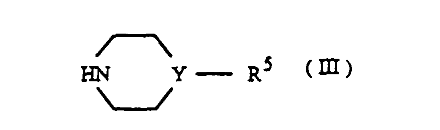 wherein Y and R⁵ have the meanings given in Claim 1, with elimination of the acid HA, and the resultant reaction product of the general formula I is converted if desired into a physiologically compatible acid addition compound.

4. Process according to Claim 3, characterised in that the reaction is in each case carried out in the presence of a base selected from the group comprising alkali and alkaline earth metal carbonates, bicarbonates, hydrides, alcoholates, hydroxides and tertiary amines.

5. Process according to Claim 3 or 4, characterised in that the reaction is carried out in the presence of a solvent inert to the reactants, from the group comprising alkanols, aromatic solvents, aliphatic and cycloaliphatic ethers, carboxylic acid dialkylamides, tetraalkyl ureas, ketones and sulphoxides.

6. Process according to one of Claims 3 to 5, characterised in that the reaction is carried out in the presence of a catalyst from the group of alkali and alkaline earth metal iodides.

7. Process according to one of Claims 3 to 6, characterised in that the reaction is carried out at a temperature between room temperature and 130°C, preferably at a temperature of less than 100°C, and at a pressure between normal pressure and 10⁷ Pa.

8. Process according to one of Claims 3 to 7, characterised in that the reaction is carried out under a protective gas atmosphere such as nitrogen or argon.

9. Pharmaceutical composition containinig at least one compound according to either Claim 1 or 2, optionally together with conventional excipients and additives.

10. Use of the compounds according to either Claim 1 or 2 to prepare a drug having neuroprotective, anticonvulsive and/or antiepileptic activity.

11. Use of the compounds according to Claim 10 to prepare a drug having in addition antidepressive, nootropic, antipsychotic and/or anxiolytic activity.

## Revendications

1. 2H-1-benzopyranne-2-ones de formule générale I, 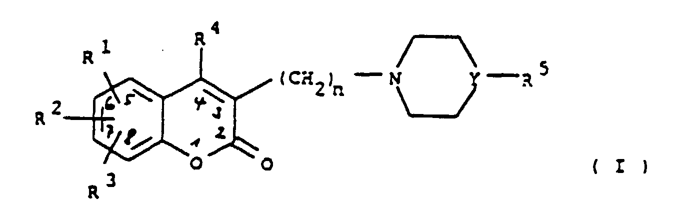 dans lesquelles
R¹ est un radical hydroxy, un radical alcoxy ayant de 1 à 5 atomes de carbone, un radical cycloalcoxy ayant de 4 à 6 atomes de carbone ou un radical alkyl- ou arylsulfonyloxy R⁶-SO₂O-,
R² et R³, indépendamment l'un de l'autre, sont des atomes d'hydrogène, des radicaux hydroxy, des radicaux alcoxy ayant de 1 à 4 atomes de carbone ou des radicaux cycloalcoxy ayant de 4 à 6 atomes de carbone,
R⁴ est un atome d'hydrogène, un groupe alkyle ayant de 1 à 4 atomes de carbone ou un groupe phényle,
Y est un atome d'azote, un groupe CH ou un groupe COH,
R⁵ est un radical phényle, naphtyle, pyridinyle ou pyrimidinyle, qui est éventuellement substitué par un ou deux groupe alkyle en C₁ à C₅, par un ou deux atomes d'halogène, par un halogène et simultanément par un groupe alkyle en C₁ à C₅, par un groupe perfluoralkyle ayant de 1 à 3 atomes de carbone, un groupe alcoxy en C₁ à C₅, un groupe hydroxy, un groupe méthylènedioxy ou nitro,
R⁶ est un radical alkyle ayant de 1 à 5 atomes de carbone, un radical cycloalkyle ayant de 4 à 6 atomes de carbone ou un radical phényle, qui éventuellement est substitué par un ou deux groupes alkyle en C₁ à C₅, par un ou deux atomes d'halogène ou par un groupe perfluoralkyle ayant de 1 à 3 atomes de carbone, et
n est un nombre entier de 1 à 4, ainsi que leurs composés d'addition avec des acides tolérés d'un point de vue physiologique, mais à l'exception de la 7,8-diméthoxy-4-méthyl-3-[(4-phényl-1-pipérazinyl)méthyl]-2H-1-benzopyranne-2-one.

2. Composés de formule I selon la revendication 1, dans lesquels :
R¹ est un radical hydroxy, méthoxy, éthoxy, propyloxy ou éthanesulfonyloxy,
R² et R³, indépendamment l'un de l'autre, sont des atomes d'hydrogène, des radicaux hydroxy ou alcoxy ayant de 1 à 3 atomes de carbone,
R⁴ est un groupe méthyle,
Y est un atome d'azote, un groupe CH ou un groupe COH,
R⁵ est un radical phényle éventuellement substitué par des substituants hydroxy, méthoxy, éthoxy, méthyle, fluoro ou trifluorométhyle, et
n = 2
ainsi que leurs composés d'addition avec des acides tolérés d'un point de vue physiologique.

3. Procédé pour préparer les composés selon la revendication 1 ou 2, caractérisé en ce qu'on fait réagir un composé de formule générale II 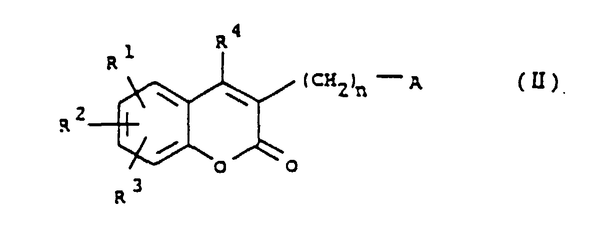 dans laquelle R¹, R², R³, R⁴ et n ont les significations données dans la revendication 1, et A est un groupe éliminable, qui est choisi parmi l'ensemble comprenant les groupes chloro, bromo, iodo, alkylsulfonyloxy, trifluorométhanesulfonyloxy, phénylsulfonyloxy éventuellement substitué par des substituants alkyle, nitro ou halogéno, avec un composé de formule générale III 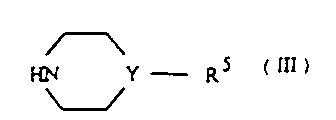 dans laquelle Y et R⁵ ont les significations données dans la revendication 1, par élimination de l'acide HA, et en ce que l'on convertit éventuellement, en un composé d'addition avec un acide toléré d'un point de vue physiologique, le produit de réaction obtenu de formule générale I.

4. Procédé selon la revendication 3, caractérisé en ce que la réaction est dans tous les cas réalisée en présence d'une base, qui est choisie dans l'ensemble comprenant les carbonates, hydrogénocarbonates, hydrures, alcoolates, hydroxydes de métaux alcalins et alcalino terreux, et les amines tertiaires.

5. Procédé selon la revendication 3 ou 4, caractérisé en ce que la réaction est mise en oeuvre en présence d'un solvant inerte vis-à-vis des substances participant à la réaction, choisis dans l'ensemble comprenant les alcanols, les solvants aromatiques, les éthers aliphatiques et cycloaliphatiques, les carboxodialkylamides, les tétraalkylurées, les cétones et les sulfoxydes.

6. Procédé selon l'une quelconque des revendications 3 à 5, caractérisé en ce que la réaction est mise en oeuvre en présence d'un catalyseur du groupe des iodures de métaux alcalins et alcalino-terreux.

7. Procédé selon l'une quelconque des revendications 3 à 6, caractérisé en ce que la réaction est mise en oeuvre à une température comprise entre la température ambiante et 130°C, de préférence à une température inférieure à 100°C, et sous une pression comprise entre la pression normale et 10⁷ Pa.

8. Procédé selon l'une quelconque des revendications 3 à 7, caractérisé en ce que la réaction est mise en oeuvre sous une atmosphère d'un gaz protecteur, tel que N₂ ou Ar.

9. Composition pharmaceutique, contenant au moins un composé la revendication 1 ou 2, éventuellement en même temps que des adjuvants et additifs usuels.

10. Utilisation des composés selon la revendication 1 ou 2 pour préparer un médicament présentant une activité neuroprotectrice, anti-convulsivante et/ou anti-épileptique.

11. Utilisation des composés selon la revendication 10 pour préparer un médicament ayant en outre une activité anti-dépressive, nootropique, antipsychotique et/ou anxiolytique.
